(19) Europäisches Patentamt
European Patent Office
Office européen des brevets

(11) Veröffentlichungsnummer: **0 102 509**
**A1**

(12) **EUROPÄISCHE PATENTANMELDUNG**

(21) Anmeldenummer: 83107368.9

(22) Anmeldetag: 27.07.83

(51) Int. Cl.³: C 07 C 103/44
C 07 C 121/78, C 07 D 213/75
A 61 K 31/16, A 61 K 31/44

(30) Priorität: 07.08.82 DE 3229539

(43) Veröffentlichungstag der Anmeldung:
14.03.84 Patentblatt 84/11

(84) Benannte Vertragsstaaten:
AT BE CH DE FR GB IT LI NL SE

(71) Anmelder: BASF Aktiengesellschaft
Carl-Bosch-Strasse 38
D-6700 Ludwigshafen(DE)

(72) Erfinder: Eilingsfeld, Heinz, Dr.
Pierstrasse 9a
D-6710 Frankenthal(DE)

(72) Erfinder: Neumann, Peter, Dr.
Franz-Schubert-Strasse 1
D-6908 Wiesloch(DE)

(72) Erfinder: Lenke, Dieter, Dr.
Kekuleplatz 1
D-6700 Ludwigshafen(DE)

(72) Erfinder: Friedrich, Ludwig, Dr.
Nibelungenstrasse 8a
D-6831 Bruehl(DE)

(54) **Neue Oxamidsäure-Derivate, Verfahren zu ihrer Herstellung und diese enthaltende therapeutische Mittel.**

(57) Oxamidsäureester der Formeln I und II

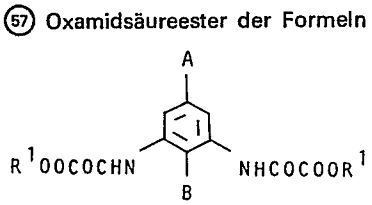 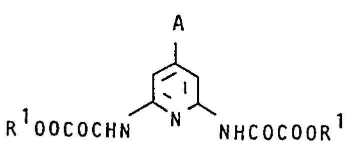,

I II

wobei A und B Wasserstoff, Chlor, Brom oder Cyan und $R^1$ einen Alkoxyethylrest mit 1 bis 4 C-Atomen im Alkoxyrest bedeuten, die bei der Behandlung allergischer Erkrankungen verwendet werden können, Verfahren zu ihrer Herstellung und diese enthaltende therapeutische Mittel.

EP 0 102 509 A1

# Neue Oxamidsäure-Derivate, Verfahren zu ihrer Herstellung und diese enthaltende therapeutische Mittel

Die Erfindung betrifft neue Oxamidsäureester, Verfahren zu ihrer Herstellung und diese enthaltende therapeutische Mittel, die bei der Behandlung allergischer Erkrankungen verwendet werden können.

Es sind eine Reihe von Derivaten der Oxamidsäure und ihrer Ester bekannt, für die eine Verwendung bei der Bekämpfung oder Unterdrückung von allergischen Reaktionen beschrieben wird. Beispielsweise sind aus der DE-OS 23 62 409, der DE-OS 25 25 164 und der DE-OS 26 37 882 Oxamidsäurederivate von Diaminobenzolen, aus der DE-OS 25 25 188 Oxamidsäurederivate von Diaminopyridinen bekannt. Ihre Wirkungen befriedigen jedoch nicht immer.

Es wurde nun gefunden, daß Oxamidsäureester der Formeln I und II

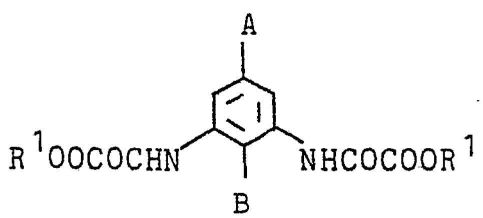

I

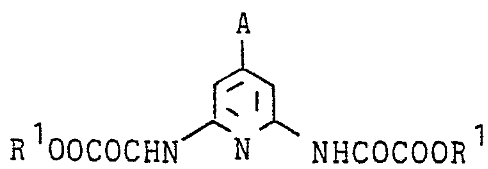 ,

II

wobei A und B Wasserstoff, Chlor, Brom oder Cyan und $R^1$ einen Alkoxyethylrest mit 1 bis 4 C-Atomen im Alkoxyrest bedeuten, wertvolle pharmakologische Eigenschaften, insbesondere als Antiallergika, aufweisen.

Die erfindungsgemäßen Verbindungen der Formeln I und II werden hergestellt, indem man Verbindungen der Formeln III und IV

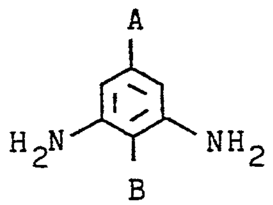

III

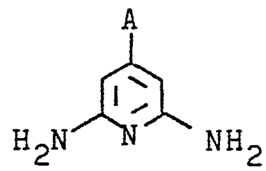

IV ,

in denen A und B die für die Formeln I und II angegebenen Bedeutungen haben, mit einem Oxalsäureesterhalogenid der Formel V

$$R^{1}O-\underset{O}{\underset{\|}{C}}-\underset{O}{\underset{\|}{C}}-Hal \qquad V,$$

in der $R^1$ die für die Formeln I und II angegebenen Bedeutungen hat und Hal ein Halogenatom bedeutet, zweckmäßig in einem aprotischen Lösungsmittel und in Anwesenheit eines Säureakzeptors in an sich bekannter Weise umsetzt.

Geeignete aprotische Lösungsmittel sind beispielsweise Methylenchlorid, Chloroform, Tetrachlorkohlenstoff, 1,2-Dichlorethan, 1,1,1-Trichlorethan, Essigsäureethylester, Dimethylformamid, Dimethylacetamid, N-Methylpyrrolidon, Tetrahydrothiophendioxid, Hexamethylphosphorsäuretriamid, Tetrahydrofuran, Dioxan oder Mischungen derselben.

Beispiele für Säureakzeptoren sind Triethylamin, Tributylamin, Pyridin. Die Reaktion zwischen den Aminen der Formeln III und IV und den Oxalsäureesterhalogeniden wird vorzugsweise bei relativ niederen Temperaturen durchgeführt, wobei Temperaturen zwischen 0 und 30°C üblich sind.

An Stelle der freien Amine kann man die Hydrochloride verwenden, wenn man ein zusätzliches Äquivalent Base einsetzt.

Eine besonders einfache Ausführungsform dieses Verfahrens besteht darin, daß man Amine der Formeln III oder IV mit einem Oxalsäureesterhalogenid der Formel V in einem inerten Lösungsmittel in Gegenwart von Polyvinylpyridin als Base umsetzt. Nach erfolgter Reaktion filtriert man von der polymeren Base bzw. ihrem Salz ab und isoliert die Oxamidsäureester der Formeln I oder II aus dem Filtrat in üblicher Weise.

Oxamidsäureester der Formeln I und II lassen sich auch nach üblichen, beispielsweise in der Schweizer Patentschrift 512 257 beschriebenen Verfahren durch Umesterung der entsprechenden Oxamidsäureniedrigalkylester mit einem Alkohol der Formel $R^1OH$, wobei $R^1$ die für die Formeln I und II angegebenen Bedeutung hat, gewinnen.

Die erfindungsgemäßen Oxamidsäureester können auch aus den freien Oxamidsäuren nach Überführung in das Oxamidsäurehalogenid, vorzugsweise -chlorid, durch Veresterung mit einem entsprechenden Alkohol $R^1OH$ in Gegenwart eines säurebindenden Mittels, wie Triethylamin, in einem aprotischen Lösungsmittel bei etwa Raumtemperatur in an sich üblicher Weise hergestellt werden.

Die Veresterung der Oxamidsäuren kann auch in Gegenwart eines sauren Katalysators oder eines Carbodiimids, wie es in Chem. Ber. 100, 16 (1967) bzw. in Acta Chem. Scand. B 33, 410 (1979) beschrieben ist, durchgeführt werden. Die Veresterung der freien Oxamidsäure kann außerdem mittels eines Dimethylformamid-acetals nach der Imidazolid-Methode

[Chem. Ber. 95, 1284 (1962)] oder nach dem in Bull. Chem. Soc. Japan 50, 1863 (1977) beschriebenen Verfahren erfolgen.

Die als Ausgangsstoffe verwendeten Diamine der allgemeinen Formeln III und IV sind bekannt oder können nach bekannten Methoden hergestellt werden.

Die erfindungsgemäßen Verbindungen und ihre physiologisch verträglichen Säureadditionssalze sind Pharmaka, die als wertvolle Hilfsmittel bei der Behandlung von allergischen Erkrankungen des Respirations-, des Gastro-Intestinaltraktes und der Haut (z.B. allergisches Asthma, allergische Rhinitis, Nahrungsmittel-Allergien usw.) verwendet werden können.

Die antiallergische Wirkung wurde an Ratten getestet. Zur Prüfung wurde das Modell der passiven cutanen Anaphylaxie (PCA) verwendet:

Narkotisierte männliche Ratten (100 - 140 g) werden durch intradermale Injektion (rasierte Rückenhaut) von 0,1 ml eines Ovalbumin-Antiserums sensibilisiert. Nach einer Sensibilisierungsdauer von ca. 48 h erfolgt die Behandlung (orale Applikation) mit unterschiedlichen Dosierungen (10 Tiere/Dosis) der Prüfsubstanzen. 15 bzw. 20 min nach der Behandlung wird eine Antigen/Evansblau-Mischung (10 mg/kg Ovalbumin in 2%iger Evansblau-Lösung) den Versuchstieren intravenös injiziert. 30 min später werden die Tiere getötet, die Rückenhaut abgezogen und auf der inneren Oberfläche der Durchmesser der kreisförmigen Blaufärbung gemessen. Die Größe der Farbflecke unbehandelter Kontrolltiere ist standardisierbar. Antiallergisch wirksame Substanzen verkleinern dosisabhängig den Durchmesser der Farbflecke. Als ED 50 % wird die Dosis angegeben, die

im Vergleich zu medikamentös unbehandelten Kontrolltieren den Durchmesser der Farbflecke um 50 % herabsetzt.

Die erfindungsgemäßen Verbindungen sind antiallergisch wirksam. Aus Tabelle 1 geht hervor, daß diese Substanzen nach oraler Gabe 1,85 (Bsp. 3) und 3,10 (Bsp. 2) mal wirksamer sind als die Vergleichsverbindung (N-Benzthiazol-2--yl-oxamidsäureester A, vgl. DOS 27 51 441; DOS 26 56 468). Die Handelssubstanz Cromolyne ist bis 100 mg/kg p.o. unwirksam.

Tabelle 1: Antiallergische Wirkung nach oraler Gabe. PCA. Ratte.

| Substanz des Beispiels Nr. | Hemmung der PCA ED 50 % mg/kg | R.W. |
|---|---|---|
| 2 | 2.77 | 3.10 |
| 3 | ca. 4.64 | ca. 1.85 |
| A | 8.60 | 1.00 |
| Cromolyne | >100 | - |

Für die therapeutische Anwendung werden Einzeldosen von 0,1 bis 100 mg pro kg Lebendgewicht verwendet.

Gegenstand der vorliegenden Erfindung sind demnach auch therapeutische Mittel oder Zubereitungen, die neben üblichen Träger- und Verdünnungsmitteln eine Verbindung der Formel I oder II als Wirkstoff enthalten, sowie die Verwendung der neuen Verbindungen bei der Herstellung von Arzneimitteln mit antiallergischer Wirkung.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Suspensionen oder Depotformen. Weiterhin kommen Inhalate und parenterale Zubereitungen, wie Injektionslösungen, in Betracht.

Die galenischen Applikationsformen, fest oder flüssig, werden in üblicher Weise hergestellt. Die Wirkstoffe können dabei mit den gebräuchlichen galenischen Hilfsmitteln (Träger, Binde- und Verdünnungsmitteln) wie Talkum, Gummi arabicum, Saccharose, Lactose, Getreide- oder Maisstärke, Kartoffelmehl, Magnesiumstearat, Alinaten, Gummi tragacanth, Carragheenate, Polyvinylalkohol, Polyvinylpyrrolidon, wäßrigen oder nicht wäßrigen Trägern, Netzmitteln, Dispergiermitteln, Emulgatoren und/oder Konservierungsmitteln, verarbeitet werden (vgl. L.G. Goodman, A. Gilman, The Pharmacological Basis of Therapeutics).

Beispiel 1

N,N'-(1,3-Phenylen)-dioxamidsäure-2-methoxyethylester

7,33 g 1,3-Phenylendiamin wurden in einem Gemisch aus 60 ml trockenem Dimethylformamid und 16,6 g Triethylamin gelöst. Unter Kühlen mit Eiswasser tropfte man 26,6 g Oxalsäure-2-methoxy-ethylesterchlorid mit einer solchen Geschwindigkeit zu, daß die Temperatur 10°C nicht überschritt. Man rührte noch eine Stunde bei einer Temperatur von 5 bis 10°C und danach etwa 20 Stunden (über Nacht) bei Raumtemperatur. Man goß auf Eiswasser, rührte das Gemisch ca. 15 Minuten, saugte den ausgefallenen Niederschlag ab und trocknete ihn auf Ton. Nach Umkristallisation aus Isopropanol/Dimethylformamid (10 : 1) erhielt man 20 g der Verbindung der Formel Ia mit A = B = C = D = H und $R^1 = CH_2CH_2OCH_3$ vom Schmp. 152-153°C.

$C_{16}H_{20}N_2O_8$ (368)

| | C | H | N | O |
|---|---|---|---|---|
| Ber. | 52,17 | 5,47 | 7,61 | 34,75 |
| Gef. | 52,3 | 5,6 | 7,7 | 34,8 |

Beispiel 2

N,N'-(1,3-Phenylen)-dioxamidsäure-2-ethoxyethylester

Aus 7,33 g 1,3-Phenylendiamin und 26,3 g Oxalsäure-2--ethoxyethylesterchlorid erhielt man beim Arbeiten analog Beispiel 1 nach Umkristallisation aus Ethanol 19,1 g der Verbindung der Formel Ia mit A = B = C = D = H und $R^1 = CH_2CH_2OC_2H_5$ vom Schmp. 122-123°C.

$C_{18}H_{24}N_2O_8$ (396)

| | C | H | N | O |
|---|---|---|---|---|
| Ber. | 54,54 | 6,10 | 7,07 | 32,29 |
| Gef. | 54,6 | 6,1 | 7,0 | 32,2 |

Beispiel 3

N,N'-(1,3-Phenylen)-dioxamidsäure-2-butoxyethylester

Aus 7,33 g 1,3-Phenylendiamin und 33,4 g Oxalsäure-2-butoxyethylesterchlorid erhielt man beim Arbeiten analog Beispiel 1 nach Umkristallisation aus Methanol 9,5 g der Verbindung der Formel Ia mit A = B = C = D = H und $R^1$ = $-C_2H_4-O-C_4H_9$ vom Schmp. 121-122°C.

$C_{12}H_{32}N_2O_8$ (453)

| | C | H | N | O |
|---|---|---|---|---|
| Ber. | 58,40 | 7,13 | 6,19 | 28,29 |
| Gef. | 58,4 | 7,1 | 6,2 | 27,9 |

Beispiel 4

N,N'-(2-Chlor-5-cyan-1,3-phenylen)-dioxamidsäure-2-methoxyethylester

Aus 5,62 g 2-Chlor-5-cyan-1,3-phenylendiamin und 13,3 g Oxalsäure-2-methoxyethylesterchlorid erhielt man beim Arbeiten analog Beispiel 1 nach Umkristallisation aus Ethanol 5,15 g der Verbindung der Formel Ia mit A = C = H, B = CN, D = Cl und $R^1$ = $CH_2CH_2OCH_3$ vom Schmp. 135-136°C.

$C_{17}H_{18}N_3O_8Cl$ (427)

| | C | H | N | O | Cl |
|---|---|---|---|---|---|
| Ber. | 47,73 | 4,24 | 9,82 | 29,92 | 8,29 |
| Gef. | 47,9 | 4,5 | 9,9 | 30,2 | 8,3 |

Beispiel 5

N,N'-(2-Chlor-5-cyan-1,3-phenylen)-dioxamidsäure-2-ethoxyethylester

Aus 5,62 g 2-Chlor-5-cyan-1,3-phenylendiamin und 14,8 g Oxalsäure-2-ethoxyethylesterchlorid erhielt man beim Arbeiten analog Beispiel 1 nach Umkristallisation aus Ethanol 8,4 g der Verbindung der Formel Ia mit A = C = H, B = CN, D = Cl und $R^1 = CH_2CH_2OC_2H_5$ vom Schmp. 105-106°C.

$C_{19}H_{22}N_3O_8Cl$ (456)

| | C | H | N | O | Cl |
|---|---|---|---|---|---|
| Ber. | 50,06 | 4,86 | 9,22 | 28,08 | 7,78 |
| Gef. | 50,2 | 4,8 | 9,4 | 27,8 | 7,8 |

Beispiel 6

N,N'-2,6-Pyridylen-bis(oxamidsäure-2-ethoxyethylester)

Aus 5,45 g 2,6-Diaminopyridin und 18,95 g Oxalsäure-2-ethoxyethylesterchlorid erhielt man beim Arbeiten analog Beispiel 1 nach Umkristallisation aus Isopropanol/Aktivkohle 3,1 g der Verbindung der Formel IIa mit A = B = C = H und $R^1 = CH_2CH_2OC_2H_5$ vom Schmp. 78-79°C.

$C_{17}H_{23}N_3O_8$ (397)

| | C | H | N | O |
|---|---|---|---|---|
| Ber. | 51,38 | 5,83 | 10,57 | 32,21 |
| Gef. | 51,6 | 5,8 | 11,1 | 32,2 |

Patentansprüche

1. Oxamidsäureester der Formeln I und II

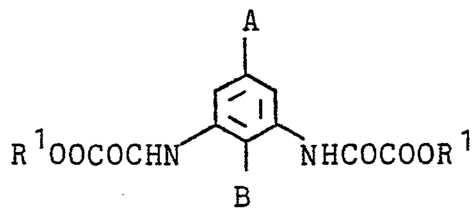 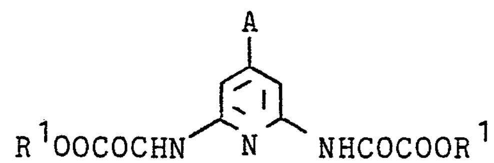 ,

I II

wobei A und B Wasserstoff, Chlor, Brom oder Cyan und $R^1$ einen Alkoxyethylrest mit 1 bis 4 C-Atomen im Alkoxyrest bedeuten.

2. Oxamidsäureester der Formel I und II nach Anspruch 1, wobei A = H oder CN und B = H oder Cl.

3. Verfahren zur Herstellung von Oxamidsäureestern nach Anspruch 1 durch Umsetzung von Verbindungen der Formeln III und IV

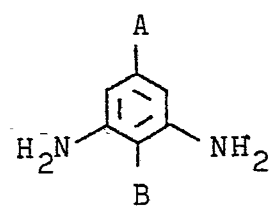 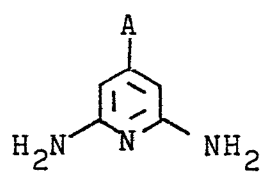 ,

III IV

in denen A und B die für die Formeln I und II in Anspruch 1 angegebenen Bedeutungen haben, mit einem Oxalsäureesterhalogenid der Formel V

$$R^1O-\underset{\underset{O}{\|}}{C}-\underset{\underset{O}{\|}}{C}-Hal \qquad V ,$$

in der $R^1$ die für die Formeln I und II in Anspruch 1

angegebenen Bedeutungen hat und Hal ein Halogenatom bedeutet.

4. Verfahren nach Anspruch 3, wobei man die Umsetzung in einem aprotischen Lösungsmittel und in Anwesenheit eines Säureakzeptors in an sich bekannter Weise durchführt.

5. Verfahren zur Herstellung von Oxamidsäureestern nach Anspruch 1 analog dem in der Schweizer Patentschrift 512 257 beschriebenen Verfahren durch Umesterung eines entsprechenden Oxamidsäureniedrigalkylesters mit einem Alkohol der Formel $R^1OH$, wobei $R^1$ die für die Formeln I und II in Anspruch 1 angegebene Bedeutung hat.

6. Verfahren zur Herstellung von Oxamidsäureestern nach Anspruch 1 aus den den Formeln I und II entsprechenden freien Oxamidsäuren nach Überführung in das Oxamidsäurechlorid durch Veresterung mit einem entsprechenden Alkohol $R^1OH$ in Gegenwart eines säurebindenden Mittels, wobei $R^1$ die für die Formeln I und II in Anspruch 1 angegebene Bedeutung hat.

7. Therapeutische Mittel oder Zubereitungen, die neben üblichen Träger- und Verdünnungsmitteln und gegebenenfalls anderen Wirkstoffen mindestens einen Oxamidsäureester nach Anspruch 1 als Wirkstoff enthalten.

8. Verfahren zur Herstellung von Arzneimitteln mit antiallergischer Wirkung, dadurch gekennzeichnet, daß man Oxamidsäureester nach Anspruch 1 in an sich üblicher Weise galenisch zubereitet.

0102509

Europäisches Patentamt

# EUROPÄISCHER RECHERCHENBERICHT

Nummer der Anmeldung

EP 83 10 7368

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,A | FR-A-2 211 230 (THE UPJOHN CY.) * Beispiele; Ansprüche * | 1-8 | C 07 C 103/44<br>C 07 C 121/78<br>C 07 D 213/75<br>A 61 K 31/16<br>A 61 K 31/44 |
| D,A | FR-A-2 273 524 (THE UPJOHN CY.) * Beispiele; Ansprüche * | 1-8 | |
| A | FR-A-2 247 237 (THE UPJOHN CY.) * Beispiele; Ansprüche * | 1-8 | |
| A | FR-A-2 389 324 (ICI) * Seiten 7,8; Verbindungen 116-118 * | 1-8 | |
| | | | **RECHERCHIERTE SACHGEBIETE (Int. Cl. 3)** |
| | | | C 07 C 103/00<br>C 07 C 121/00<br>C 07 D 213/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 10-11-1983 | PAUWELS G.R.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82